# EUROPÄISCHE PATENTSCHRIFT

(11) **EP 1 837 008 B1**
(45) Veröffentlichungstag und Bekanntmachung des Hinweises auf die Patenterteilung: **08.10.2008**
(21) Anmeldenummer: 07104519.9
(22) Anmeldetag: 20.03.2007
(51) Int. Cl.: A61K 8/04, A61Q 19/08

(54) **Kosmetikum zur nachhaltigen Behandlung tieferer Hautfalten**
Cosmetic agent for lasting treatment of deeper wrinkles in the skin
Produit cosmétique destiné au traitement durable des rides profondes

(30) Priorität: 21.03.2006 DE 102006013925
(43) Veröffentlichungstag der Anmeldung: 26.09.2007
(73) Patentinhaber: Coty Prestige Lancaster Group GmbH, 55116 Mainz (DE)
(72) Erfinder: Golz-Berner, Karin, 98000, Monaco (MC); Zastrow, Professor Leonhard, 98000 Monaco (MC)
(74) Vertreter: Ziebig, Marlene

(56) Entgegenhaltungen:
- EP-A- 1 634 625
- WO-A-92/12702
- FR-A1- 2 828 399
- BAUZA E ET AL: "DATE PALM KERNEL EXTRACT EXHIBITS ANTIAGING PROPERTIES AND SIGNIFICANTLY REDUCES SKIN WRINKLES" INTERNATIONAL JOURNAL OF TISSUE REACTIONS, BIOSCIENCE EDIPRINT, GENEVA, CH, Bd. 24, Nr. 4, 2002, Seiten 131-136, XP009057828 ISSN: 0250-0868

## Beschreibung

Die Erfindung betrifft ein Kosmetikum, das zur nachhaltigen Behandlung tieferer Hautfalten eingesetzt werden kann. Es umfasst Mikrokügelchen auf Basis von silyliertem Siliziumdioxid mit Weizenproteinen, Mikrokügelchen auf Basis von silyliertem Siliziumdioxid mit Hyaluronsäure, in Chitosan verkapseltes Retinol und Dattelsamenextrakt, zusammen in einer Kombination aus leichtflüchtigen Silikonölen und in Wasser nicht löslichen Silikonpolymeren, wobei die Silikonpolymeren wenigstens ein vernetztes Silikonpolymeres mit endständigen Vinylgruppen enthalten. Die Wirkstoffkonzentrationen in der Assoziation sind sehr gering, und das Kosmetikum zeigt einen Langzeiteffekt bei der Rückbildung von Gesichtshautfalten, der überraschend schnell auftritt, nahezu konstant bleibt und sehr spät langsam abfällt.

Kosmetische Zusammensetzungen mit Anti-Faltenwirkung oder Anti-Alterungwirkung sind bereits bekannt. Sie enthalten oftmals Vitamin E sowie weitere radikalfangende Substanzen. So ist aus der US2002/0058076A1 beispielsweise ein hydrolysiertes Weizenprotein bekannt, das sich als Anti-Alterungsmittel für die Haut einsetzen lässt. Auch andere Pflanzenextrakte sowie Hyaluronsäure sind für diesen Zweck bereits eingesetzt worden.

Die DE 10 2004 39 631 offenbart ein Anti-Alterungsmittel aus Alfalfa-Extrakt, Meerfenchel-Extrakt, Retinol/Retinylpalmitat und Schichtpigmenten.

Die nachhaltige Wirkung, insbesondere bei tieferen Hautfalten, ist jedoch bei bekannten Anti-Alterungsmitteln nicht immer befriedigend.

Aufgabe der Erfindung ist es daher, ein Kosmetikum bereitzustellen, das eine schnelle Wirksamkeit auch bei tieferen Hautfalten zeigt und diese Wirkung auch über längere Zeiträume aufrechterhält.

Das erfindungsgemäße Kosmetikum ist gekennzeichnet durch eine Kombination umfassend Mikrokügelchen auf Basis von silyliertem Siliziumdioxid mit Weizenproteinen, Mikrokügelchen auf Basis von silyliertem Siliziumdioxid mit Hyaluronsäure, in Chitosan verkapseltes Retinol und Dattelsamenextrakt, zusammen in einer Kombination aus leichtflüchtigen Silikonölen und in Wasser nicht löslichen Silikonpolymeren, wobei die Silikonpolymeren wenigstens ein vernetztes Silikonpolymeres mit endständigen Vinylgruppen enthalten.

Insbesondere sind die Mikrokügelchen auf Basis von silyliertem Siliziumdioxid in einer Konzentration von 0,00025-0,05 Gew.-%, bezogen auf das Gesamtgewicht des Kosmetikums, enthalten. Die Mikrokügelchen auf Basis von silyliertem Siliziumdioxid mit Hyaluronsäure oder einem Hyaluronsäuresalz sind bevorzugt in einer Konzentration von 0,0001-0,003 Gew.-%, bezogen auf das Gesamtgewicht des Kosmetikums, enthalten. Das in Chitosan verkapselte Retinol ist vorzugsweise zu 0,002-0,015 Gew.-% im Kosmetikum enthalten. Die Konzentration des Dattelsamenextraktes im Kosmetikum beträgt bevorzugt 0,0005-0,05 Gew.-%. Der Anteil der leichtflüchtigen Silikonöle im Kosmetikum beträgt bevorzugt 13-28 Gew. -%, und der Anteil der in Wasser nicht löslichen Silikonpolymere beträgt bevorzugt 1,5-15 Gew.-%. Das Verhältnis leichtflüchtiger Silikonöle zu Silikonpolymeren liegt vorzugsweise im Bereich von 3,5 bis 6 : 1. Der Rest zu 100 Gew. -% wird durch kosmetische Hilfsstoffe, Trägerstoffe, gegebenenfalls weitere Aktivstoffe oder Gemische davon gebildet.

In einer bevorzugten Ausführungsform ist das erfindungsgemäße Kosmetikum gekennzeichnet durch eine Assoziation, bestehend aus
0,00025 bis 0,05 Gew.-% Mikrokügelchen auf Basis von silyliertem Siliziumdioxid mit Weizenproteinen,
0,0001 bis 0,003 Gew.-% Mikrokügelchen auf Basis von silyliertem Siliziumdioxid mit Hyaluronsäure oder einem Hyaluronsäuresalz,
0,002 bis 0,015 Gew.-% eines in Chitosan verkapselten Retinols und
0,0005 bis 0,05 Gew.-% Dattelsamenextrakt
in einer Kombination aus 13 bis 28 Gew.-% leichtflüchtigen Silikonölen und 1,5 bis 15 Gew.-% in Wasser nicht löslichen Silikonpolymeren, wobei die Silikonpolymeren wenigstens ein vernetztes Silikonpolymeres mit endständigen Vinylgruppen enthalten, und das Verhältnis leichtflüchtiger Silikonöle zu Silikonpolymeren im Bereich von 3,5 bis 6:1 liegt. Der Rest zu 100 Gew.-% wird durch kosmetische Hilfsstoffe, Trägerstoffe, gegebenenfalls weitere Aktivstoffe oder Gemische davon gebildet. Alle Gewichtsangaben sind auf das Gesamtgewicht des Kosmetikums bezogen.

Das erfindungsgemäße Kosmetikum enthält in einer vorteilhaften Ausführungsform 0,1 bis 1,2 Gew.-% Siliziumdioxidkügelchen mit einer Teilchengröße D₅₀ im Bereich von 3,5 bis 8 µm. Die Teilchengröße wird mit einem Malvern®-Gerät bestimmt. D₅₀ ist eine mittlere Teilchengröße und bedeutet, dass wenigstens 50% der Teilchen in dem angegebenen Bereich liegen.

Die Mikrokügelchen auf Basis von silyliertem Siliziumdioxid sind dehydratisierte Mikrokügelchen und vorteilhaft solche des Typs mit der INCI-Bezeichung Silica Dimethyl Silylate.

Das Gewichtsverhältnis des silylierten Siliziumdioxids zu dem Weizenprotein liegt vorzugsweise im Bereich 1 bis 3:1.

Das Gewichtsverhältnis des silylierten Siliziumdioxids zu der Hyaluronsäure oder ihrem Alkalisalz liegt vorzugsweise im Bereich von 8 bis 16:1.

Das leichtflüchtige Silikonöl ist vorzugsweise ein Gemisch aus Polydimethylsiloxan mit einer Viskosität von 9-11 mPa·s sowie Cylclopentasiloxan und Cyclohexasiloxan mit einer Viskosität von 4-8 mPa·s.

Unter "leichtflüchtiges Silikonöl" werden solche Silikonöle verstanden, deren Verdampfungswärme im Bereich von 150 bis 200 kJ/kg bei 25 °C liegt und deren Viskosität meist unter 10 mPa·s liegt.

Unter "Silikonpolymere" werden solche Silikonverbindungen verstanden, deren Verdampfungswärme > 200 kJ/kg beträgt und deren Viskosität bei wenigstens 700 bis 2500 mPa·s liegt (Brookfield Viskosimeter bei 25 °C). Diese Silikonpolymeren, wie z.B. Dimethicone & Dimethicone/Vinyl Dimethicone Crosspolymer, PEG-9 Polydimethylsiloxyethyl Dimethicone Cyclotetrasiloxane, Dimethicone & PEG-10 Dimethicone Crosspolymer & PEG-15 Dimethicone Crosspolymer, Cetyl Dimethicone Copolyol werden vorteilhaft bei niedrigen Rührgeschwindigkeiten im Bereich von 500 bis 1300 U/min und bei Raumtemperatur verarbeitet, um die ungleichmäßige Struktur im Inneren des Polymersystems zu erhalten und damit die Stabilität der Gel-Emulsion als Endprodukt zu gewährleisten.

Überraschenderweise zeigt der Einsatz von Retinol, z.B. als Retinol Primapheres L2, und Dattelextrakt nicht nur eine gewisse zu erwartende Anti-Alterungswirkung, sondern die Kombination mit silyliertem Siliziumdioxid mit Weizenproteinen bzw. Hyaluronsäure und den Silikonpolymeren führt schon bei sehr geringen Konzentrationen des silylierten Siliziumdioxids mit Weizenproteinen bzw. Hyaluronsäure zu einer unerwarteten Langzeitwirkung für die Rückbildung tieferer Hautfalten. Die Ursachen dafür sind bisher noch nicht erkannt worden. Ohne an eine Theorie gebunden zu sein, scheinen sich die Mikrokügelchen mit Hyaluronsäure in die unterste Ebene der Hautfalten abzusetzen und darüber die Mikrokügelchen mit Weizenproteinen. In einer vorteilhaften Ausführungsform könnten sich größere Siliziumdioxidkügelchen noch darüber abgelagert haben.

Abgesehen von dem Auffülleffekt für die Hautfalten und auch durch die Volumenzunahme der dehydratisierten Mikrokügelchen, könnte das Silikonpolymere oder -polymergemisch zu einer längeren Verweildauer der Mikrokügelchen beitragen. In jedem Fall zeigt das erfindungsgemäße Kosmetikum einen Langzeiteffekt, der überraschend schnell auftritt, nahezu konstant bleibt und sehr spät langsam abfällt. Dieser Effekt bei mittleren Faltentiefen wird durch Vergleichsversuche nachgewiesen.

Darüber hinaus trägt das Verhältnis polymerer Silikone zu leichtflüchtigen Silikonölen mit sehr niedriger Viskosität zu dem beschriebenen Langzeiteffekt bei und gleichzeitig zu einem besonders nachhaltigen und bereits unmittelbar beim Auftragen empfundenen Weichheitsgefühl.

Besonders hervorzuheben sind die sehr geringen Konzentrationen der Wirkstoffe, die in diesen Größenordnungen in der oben genannten Assoziation ihre überraschend schnelle und lange Wirkung entfalten.

Das Kosmetikum kann weiterhin bekannte Hilfs- und Trägerstoffe, wie sie üblicherweise in solchen Zubereitungen verwendet werden, enthalten, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, geringe Mengen an weiteren polaren und unpolaren Ölen, Polymere, Copolymere, Siliconemulgatoren, Siliconwachse, Stabilisatoren und Gemische davon. Die Zugabe von Estern, Gelbildnern und weiteren Ölen ist nicht bevorzugt.

Bevorzugt ist die Zugabe von 5 bis 10 Gew.-% eines mehrwertigen Alkohols, wie z.B. Propylenglycol, Butylenglycol oder Gemische davon, sowie die Zugabe von bevorzugt 1-4 Gew.-% eines einwertigen Alkohols, wie z.B. Ethanol oder Isopropanol.

Das Kosmetikum kann auch weitere Wirkstoffe enthalten. Dazu gehören z. B. anorganische und organische Lichtschutzmittel, Selbstbräunungsmittel, Radikalfänger, Vitamine, Enzyme, pflanzliche Wirkstoffe, Polymere, Antioxidationsmittel, mit Sauerstoff beladene asymmetrische lamellare Aggregate gemäß WO 94/00109; Aufschlussprodukte von Hefen oder pflanzlichen Stoffen, hergestellt durch ein schonendes Ultraschall-Aufschlussverfahren gemäß WO 94/13783, Kaolin sowie mit SiO₂ modifiziertes Kaolin gemäß WO 94/17588.

Zu Antioxidationsmitteln gehören Vitamine wie Vitamin C und Derivate davon, beispielsweise Ascorbylacetat, -phosphat und -palmitat, Magnesiumascorbylphosphat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Imidazole wie z.B. cis- oder trans-Urocaninsäure und deren Derivate; Peptide wie D,L-Carnosin, D-Carnosin, L-Carnosin und deren Derivate; Carotinoide und Carotine, wie z.B. α-Carotin, β-Carotin, Lycopin; Harnsäure und Derivate davon; α-Hydroxysäuren wie Citronensäure, Milchsäure, Apfelsäure; α-Hydroxyfettsäuren wie Palmitinsäure, Phytinsäure, Lactoferrin; Stilbene und deren Derivate; Mannose und deren Derivate; Liponsäure und deren Derivate, z.B. Dihydroliponsäure; Ferulasäure und deren Derivate; Thiole wie Glutathion, Cystein, Cystin und deren Ester; Folsäure und deren Derivate.

Bevorzugte Antioxidationsmittel bzw. Radikalfänger sind Pflanzenextrakte, insbesondere solche, die aus der Gruppe ausgewählt sind, bestehend aus Angelica archangelica, Arnica montana, Camellia sinensis, Cupressus semper, Coffee arabica, Polygonatum multiflorum, Pongamia pinnata, Rosmarinus officinalis, Evernia fufuracea, Evernia prunastri, Aventa sativa und Gemische davon.

Als Radikalfänger sind speziell bevorzugt Vitamin A, Vitamin E, Peptide, Flavone, Flavonole sowie Pflanzenextrakte. Ein besonders vorteilhafter Pflanzenextrakt ist ein solcher aus getrockneten Extrakten von Angelica archangelica, Camellia sinensis, Coffee arabica und Pongamia pinnata in einem einwertigen Alkohol.

Bestimmte Wirkstoffe können auch in Liposomen eingeschlossen werde, die durch Phospholipide gebildet werden. Als Phospholipide können z.B. eingesetzt werden Phosphatidylcholin, Phosphatidylethanolamin, Phosphatidylinositol, Phosphatidylserin, Phosphatidsäure und Lysolecithine sowie Gemische davon. Bekannte Produkte sind beispielsweise Phoslipon® oder NAT®.

Ein weiterer vorteilhafter Wirkstoff ist Ceramid 2 zusammen mit einem Oligopeptid wie Pal-Val-Gly-Val-Ala-Pro-Gly.

Dem erfindungsgemäßen Kosmetikum können auch entsprechende wasser- und/oder öllösliche UVA- oder UVB-Filter oder beide zugesetzt werden, allerdings vorzugsweise nur bis zur Erreichung eines Sonnenschutzfaktors SPF 8 (Bestimmung gemäß Colipa-Standard (Colipa-Ref.: 94/289 (1994)). Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin können auch Breitbandfilter eingesetzt werden, wie z.B. Bis-Resorcinyltriazin-Derivate, oder auch Benzoxazole.

Das erfindungsgemäße Kosmetikum liegt vorzugsweise als eine Silicon/Gel-Emulsion vor, d.h. als ein in der Ölphase ohne zusätzlichen Gelbildner gebildetes Gel.

Dem Kosmetikum zugesetzte Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titandioxid, Glimmer, Kaolin, manganhaltige Tone, Calciumcarbonat, Talkum, Glimmer-Titanoxid, Glimmer-Titanoxid-Eisenoxid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken.

Als weitere Wirkstoffe geeignete Substanzen sind Selbstbräunungsmittel, wie z.B. Isatin, Alloxan, Ninhydrin, Glycerinaldehyd, meso-Weinsäurealdehyd, Glutaraldehyd, Erythrulose, Pirazolin-4,5-dionderivate, Dihydroxyaceton (DHA), 4,4-Dihydroxypirazolin-5-dionderivate, Glyccyhriza glabra (Süßholzwurzel), Arbutin.

Die Erfindung soll nachstehend durch Beispiele näher erläutert werden. Alle Angaben erfolgen in Gewichtsprozent, sofern nichts anderes angegeben ist.

### Beispiel 1 W/O-Emulsion I für Augen und Lippen

| **Phase A** | % |
|---|---|
| Dimethicone & Cyclotetrasiloxane | 5,0 |
| Cyclopentasiloxane & Cyclohexasiloxane & Cyclotetrasiloxane | 1,5 |
| Dimethicone & Dimethicone/Vinyl Dimethicone Crosspolymer | 1,0 |
| PEG-9 Polydimethylsiloxyethyl Dimethicone Cyclotetrasiloxane | 0,5 |
| Dimethicone & PEG-10 Dimethicone Crosspolymer & PEG-15 Dimethicone Crosspolymer | 2,0 |
| Dattelsamenextrakt | 0,004 |
| Parfüm | 0,5 |
| Konservierungsmittel | 1,0 |

| **Phase B** | |
|---|---|
| Wasser | q.s. ad 100 |
| Farbstoffe | 0,3 |
| Butylenglycol | 7,0 |
| Propylenglycol | 12,0 |
| Silica | 0,15 |
| Silica Dimethyl Silylate & hydrolys. Weizenprotein | 0,0003 |
| Silica Dimethyl Silylate & Natriumhyaluronat | 0,0003 |
| Retinol Primaspheres L2® | 0,6 |
| Natriumchlorid | 0,2 |
| Polymethylmethacrylat | 0,5 |

Die Silikonöle und Silikonpolymere der Phase A werden bei Raumtemperatur, also etwa 18-25 °C, miteinander vermischt und bei 1000 U/min gut verrührt. Danach werden die Konservierungsmittel und die restlichen Bestandteile der Phase A hinzugegeben, wieder gut verrührt und anschließend das Parfümöl hinzugesetzt. Ebenfalls bei Raumtemperatur werden von Phase B Farbstoffe und Butylenglycol sowie Propylenglycol in Wasser verrührt und danach die weiteren Bestandteile der Phase B. Schließlich wird die Phase B zur Phase A hinzugegeben, vermischt, und das Gemisch wird zum Schluss nochmals wenigstens 3 Minuten bei 900 U/min gerührt.

### Beispiel 2 W/O-Emulsion II für Augen und Lippen

| **Phase A** | % |
|---|---|
| Dimethicone & Cyclotetrasiloxane | 5,0 |
| Cyclopentasiloxane & Cyclohexasiloxane & Cyclotetrasiloxane | 1,5 |
| Dimethicone & Dimethicone/Vinyl Dimethicone Crosspolymer | 1,0 |
| PEG-9 Polydimethylsiloxyethyl Dimethicone Cyclotetrasiloxane | 0,5 |
| Dimeticone & PEG-10 Dimethicone Crosspolymer & PEG-15 Dimethicone Crosspolymer | 2,0 |
| Dattelsamenextrakt | 0,004 |
| Ceramid 2 & Palmitoyloligopeptide | 0,5 |
| Parfüm | 0,5 |
| Konservierungsmittel | 1,0 |

| **Phase B** | |
|---|---|
| Wasser | q.s. ad 100 |
| Farbstoffe | 0,3 |
| Butylenglycol | 8,0 |
| Propylenglycol | 15,0 |
| Silica | 0,15 |
| Silica Dimethyl Silylate & hydrolys. Weizenprotein | 0,002 |
| Silica Dimethyl Silylate & Natriumhyaluronat | 0,0015 |
| Retinol Primaspheres L2® | 0,9 |
| Phytotonine® | 0,5 |
| Avena sativa Extrakt | 0,1 |
| Radikalfängerkomplex (RPF) II* | 0,6 |
| Natriumchlorid | 0,2 |
| Polymethylmethacrylat | 0,5 |

| | |
|---|---|
| *bestehend aus getrockneten Extrakten von Angelica archangelica, Camellia sinensis, Coffee arabica und Pongamia pinnata (jeweils 0,2%) in einem einwertigen Alkohol (99,2%). | |

Die Verfahrensweise entspricht der von Beispiel 1. Man erhält ein Gelprodukt mit äußerst angenehmem Weichheitsgefühl beim Auftragen auf die Haut.

### Beispiel 3 W/O-Emulsion III für Serum

| **Phase A** | **%** |
|---|---|
| Dimethicone & Cyclotetrasiloxane | 3,0 |
| Cyclopentasiloxane & Cyclohexasiloxane & Cyclotetrasiloxane | 2,5 |
| Dimethicone & Dimethicone/Vinyl Dimethicone Crosspolymer | 2,7 |
| Cetyl Dimethicone Copolyol | 1,0 |
| Dattelsamenextrakt | 0,001 |
| Ceramid 2 & Palmitoyloligopeptide | 0,3 |
| Parfüm | 0,3 |
| Konservierungsmittel | 0,9 |

| **Phase B** | |
|---|---|
| Wasser | q.s. ad 100 |
| Farbstoffe | 0,3 |
| Butylenglycol | 15,0 |
| Propylenglycol | 17,0 |
| Glycerin | 5,0 |
| Silica | 0,15 |
| Silica Dimethyl Silylate & hydrolys. Weizenprotein | 0,02 |
| Silica Dimethyl Silylate & Natriumhyaluronat | 0,005 |
| Retinol Primaspheres L2® | 0,6 |
| Phytotonine® | 0,1 |
| Avena sativa Extrakt | 0,5 |
| Radikalfängerkomplex (RPF) I** | 0,7 |
| Natriumchlorid | 0,1 |
| Polymethylmethacrylat | 0,01 |

| | |
|---|---|
| **bestehend aus getrockneten Extrakten von Angelica archangelica, Camellia sinensis, Coffee arabica und Pongamia pinnata (jeweils 0,2% bezogen auf den Komplex) sowie Ascorbylpalmitat, Tocopherol, Ascorbinsüre, Evernia furfuracea-Extrakt, Evernia prunastri-Extrakt in wässrigalkoholischem Medium mit Phospholipiden. | |

Die Verfahrensweise entspricht der von Beispiel 1.

### Beispiel 4 (Vergleichsbeispiel 1) Anwender-Test

Ein Anwendertest mit 50 Teilnehmerinnen im Alter von 33 bis 55 Jahren wurde durchgeführt. 25 Teilnehmerinnen (Gruppe A) applizierten zweimal täglich eine Creme gemäß Beispiel 1 um Augen- und Mundpartie des Gesichts. Die andere Gruppe (B) applizierten eine Creme gemäß Beispiel 1 jedoch ohne die Bestandteile
Dimethicone & Dimethicone/Vinyl Dimethicone Crosspolymer,
PEG-9 Polydimethylsiloxyethyl Dimethicone & Cyclotetrasiloxane, Dimethicone & PEG-10 Dimethicone Crosspolymer & PEG-15 Dimethicone Crosspolymer, Silica Dimethyl Silylate & hydrolysiertes Weizenprotein, Silica Dimethyl Silylate & Natriumhyaluronat. Dafür wurde der Anteil an Wasser erhöht (Beispiel 1a).

In den Abständen 3 Tage, 1 Woche, 2 Wochen, 4 Wochen, 6 Wochen, 8 Wochen, 12 Wochen wurden digitalisierte Aufnahmen, insbesondere vom Augenwinkel der Teilnehmerinnen, gemacht und über ein Computerprogramm nach Anzahl der Falten und Faltentiefe ausgewertet.

Die Ergebnisse sind in Tabelle 1 dargestellt.

**Tabelle 1**

| | | Faltenreduzierung [%] |
|---|---|---|
| | Gruppe A | Gruppe B2 |
| Behandlungsbeginn | 0 | 0 |
| nach 3 Tagen | 9 | 0 |
| nach 1 Woche | 14-15 | 1-2 |
| nach 2 Wochen | 19-21 | 8-11 |
| 4 Wochen | 28-32 | 10-14 |
| 6 Wochen | 33-36 | 12-14 |
| 8 Wochen | 39-43 | 14-15 |
| 10 Wochen | 42-48 | 12-14 |
| 12 Wochen | 49-56 | 16-18 |

Tabelle 1 zeigt deutlich die Überlegenheit der Augen/Lippencreme mit der erfindungsgemäßen Assoziation (A) gegenüber einer alleinigen Anwendung von bekannter Anti-Alterungssubstanzen wie Retinol und Dattelextrakt (B) gemäß Beispiel 1a.

Weitere Verbesserungen wurden mit Zusammensetzungen gemäß Beispiel 2 und 3 erzielt.

Untersuchungen nach 15 und 20 Wochen zeigen nur einen langsamen Rückgang der Faltenreduzierung nach Absetzen der Behandlung für die Gruppe A.

### Beispiel 5 (Vergleichsbeispiel 2)

In einem Anwendertest mit 12 Probandinnen im Alter von 25 bis 42 Jahren mit Mischhaut wurden Emulsionen gemäß Beispiel 1 und Beispiel 1a in einer Menge von etwa 2 mg/cm² aufgetragen. Bewertet wurden auf einer Skala von 1 bis 4 die Textur der Creme (Gleiten des Cremekörpers zwischen den Fingern) (1 = sehr leichtes Gleiten, 2 = leichtes Gleiten, 3 = geringe Widerstände beim Gleiten) sowie die Weichheit beim Verteilen auf der Haut (1 = sehr weich, 2 = weich, 3 = geringe Verteilungsschwierigkeiten). Die Emulsion nach Beispiel 1a erhielt für die Textur die Durchschnittsnote 2,3, die Emulsion nach Beispiel 1 die Durchschnittsnote 1,5. Für die Weichheit wurde die Durchschnittsnote 1,8 für Beispiel 1a und 1,2 für Beispiel 1 erreicht. Diese Ergebnisse zeigen die Überlegenheit der erfindungsgemäßen Creme gemäß Beispiel 1.

## Patentansprüche

1. Kosmetikum zur nachhaltigen Behandlung tieferer Hautfalten umfassend Mikrokügelchen auf Basis von silyliertem Siliziumdioxid mit Weizenproteinen, Mikrokügelchen auf Basis von silyliertem Siliziumdioxid mit Hyaluronsäure, in Chitosan verkapseltes Retinol und Dattelsamenextrakt, zusammen in einer Kombination aus leichtflüchtigen Silikonölen und in Wasser nicht löslichen Silikonpolymeren, wobei die Silikonpolymeren wenigstens ein vernetztes Silikonpolymeres mit endständigen Vinylgruppen enthalten.

2. Kosmetikum nach Anspruch 1, **gekennzeichnet durch** eine Assoziation, bestehend aus
0,00025 bis 0,05 Gew.-% Mikrokügelchen auf Basis von silyliertem Siliziumdioxid mit Weizenproteinen,
0,0001 bis 0,003 Gew.-% Mikrokügelchen auf Basis von silyliertem Siliziumdioxid mit Hyaluronsäure oder einen Hyaluronsäuresalz,
0,002 bis 0,015 Gew.-% eines in Chitosan verkapselten Retinols und
0,0005 bis 0,05 Gew.-% Dattelsamenextrakt
in einer Kombination aus 13 bis 28 Gew.-% leichtflüchtigen Silikonölen und
1,5 bis 15 Gew.-% in Wasser nicht löslichen Silikonpolymeren, wobei die Silikonpolymeren wenigstens ein vernetztes Silikonpolymeres mit endständigen Vinylgruppen enthalten, und das Verhältnis leichtflüchtiger Silikonöle zu Silikonpolymeren im Bereich von 3,5 bis 6:1 liegt, und der Rest zu 100 Gew.-% **durch** kosmetische Hilfsstoffe, Trägerstoffe oder Gemische davon gebildet wird, wobei alle Gewichtsangaben auf das Gesamtgewicht des Kosmetikums bezogen sind.

3. Kosmetikum nach Anspruch 1 oder 2, **dadurch gekennzeichnet, dass** es Siliziumdioxidkügelchen mit einer Teilchengröße D₅₀ im Bereich von 3,5 bis 8 µm enthält.

4. Kosmetikum nach einem der Ansprüche 1 bis 3, **dadurch gekennzeichnet, dass** die Mikrokügelchen auf Basis von silyliertem Siliziumdioxid dehydratisierte Mikrokügelchen sind des Typs Silica Dimethyl Silylate.

5. Kosmetikum nach einem der Ansprüche 1 bis 4, **dadurch gekennzeichnet, dass** das Gewichtsverhältnis des silylierten Siliziumdioxids zu dem Weizenprotein im Bereich 1 bis 3:1 liegt.

6. Kosmetikum nach einem der Ansprüche 1 bis 4, **dadurch gekennzeichnet, dass** das Gewichtsverhältnis des silylierten Siliziumdioxids zu der Hyaluronsäure oder ihrem Alkalisalz im Bereich von 8 bis 16:1 liegt.

7. Kosmetikum nach einem der Ansprüche 1 bis 6, **dadurch gekennzeichnet, dass** es 5 bis 10 Ges.-% eines mehrwertigen Alkohols enthält.

8. Kosmetikum nach einem der Ansprüche 1 bis 7, **dadurch gekennzeichnet, dass** es einen Radikalfänger oder ein Gemisch von Radikalfängern enthält.

9. Kosmetikum nach Anspruch 8, **dadurch gekennzeichnet, dass** das Gemisch von Radikalfängern in Form von Pflanzenextrakten vorliegt.

10. Kosmetikum nach Anspruch 9, **dadurch gekennzeichnet, dass** die Pflanzenextrakte aus der Gruppe ausgewählt sind, bestehend aus Angelica archangelica, Arnica montana, Camellia sinensis, Cupressus semper, Coffee arabica, Polygonatum multiflorum, Pongamia pinnata, Rosmarinus officinalis, Evernia fufuracea, Evernia prunastri, Aventa sativa und Gemische davon.

11. Kosmetikum nach einem der Ansprüche 1 bis 10, **dadurch gekennzeichnet, dass** es ein Oligopeptid enthält, insbesondere Ceramid 2.

## Claims

1. A cosmetic for the lasting treatment of fairly deep wrinkles, said cosmetic comprising microbeads based on silylated silica with wheat proteins, microbeads based on silylated silica with hyaluronic acid, chitosan-encapsulated retinol and date seed extract, together in a combination of volatile silicone oils and water-insoluble silicone polymers, said silicone polymers including at least one crosslinked silicone polymer with terminal vinyl groups.

2. The cosmetic according to claim 1, **characterized by** an association consisting of
0.00025 to 0.05 wt.% of microbeads based on silylated silica with wheat proteins,
0.0001 to 0.003 wt.% of microbeads based on silylated silica with hyaluronic acid or a hyaluronic acid salt,
0.002 to 0.015 wt.% of a chitosan-encapsulated retinol, and
0.0005 to 0.05 wt.% date seed extract,
in a combination of 13 to 28 wt.% of volatile silicone oils, and
1.5 to 15 wt.% of water-insoluble silicone polymers, said silicone polymers including at least one crosslinked silicone polymer with terminal vinyl groups, the ratio of volatile silicone oils to silicone polymers ranging from 3.5 to 6:1, and the balance to make 100 wt.% being constituted by cosmetic adjuvants, vehicles or mixtures thereof, wherein all weight-related figures are based on the overall weight of the cosmetic.

3. The cosmetic according to claim 1 or 2, **characterized in that** it includes silica beads with a particle size D₅₀ in the range of from 3.5 to 8 µm.

4. The cosmetic according to any of claims 1 to 3, **characterized in that** the microbeads based on silylated silica are dehydrated microbeads of the silica dimethyl silylate type.

5. The cosmetic according to any of claims 1 to 4, **characterized in that** the weight ratio of silylated silica to wheat protein is in the range of from 1 to 3:1.

6. The cosmetic according to any of claims 1 to 4, **characterized in that** the weight ratio of silylated silica to hyaluronic acid or alkali salt thereof is in the range of from 8 to 16:1.

7. The cosmetic according to any of claims 1 to 6, **characterized in that** it includes 5 to 10 wt.% of a polyhydric alcohol.

8. The cosmetic according to any of claims 1 to 7, **characterized in that** it includes a free-radical scavenger or a mixture of free-radical scavengers.

9. The cosmetic according to claim 8, **characterized in that** the mixture of free-radical scavengers is present in the form of plant extracts.

10. The cosmetic according to claim 9, **characterized in that** the plant extracts are selected from the group consisting of Angelica archangelica, Arnica montana, Camellia sinensis, Cupressus semper, Coffea arabica, Polygonatum multiflorum, Pongamia pinnata, Rosmarinus officinalis, Evernia furfuracea, Evernia prunastri, Avena sativa and mixtures thereof.

11. The cosmetic according to any of claims 1 to 10, **characterized in that** it includes an oligopeptide, particularly ceramide 2.

## Revendications

1. Produit cosmétique destiné au traitement durable des plis cutanés profonds comprenant des microbilles à base de dioxyde de silicium silylé avec des protéines de blé, des microbilles à base de dioxyde de silicium silylé avec de l'acide hyaluronique, du rétinol encapsulé dans du chitosane et de l'extrait de graine de dattier, ensemble avec une combinaison d'huiles de silicone très volatiles et de polymères de silicone insolubles dans l'eau, moyennant quoi les polymères de silicone contiennent au moins un polymère de silicone réticulé comportant des groupes vinyle terminaux.

2. Produit cosmétique selon la revendication 1, **caractérisé par** une association constituée de
0,00025 à 0,05 % en poids de microbilles à base de dioxyde de silicium silylé avec des protéines de blé,
0,0001 à 0,003 % en poids de microbilles à base de dioxyde de silicium silylé avec de l'acide hyaluronique ou un sel d'acide hyaluronique,
0,002 à 0,015 % en poids d'un rétinol encapsulé dans du chitosane et
0,0005 à 0,05 % en poids d'extrait de graine de dattier,
dans une combinaison de 13 à 28% en poids d'huiles de silicone très volatiles et
1,5 à 15 % en poids de polymères de silicone insolubles dans l'eau, moyennant quoi les polymères de silicone contiennent au moins un polymère de silicone réticulé comportant des groupes vinyles terminaux, et le rapport des huiles de silicone très volatiles aux polymères de silicone est dans une plage de 3,5 à 6 : 1, le reste pour compléter à 100 % en poids étant constitué de produits auxiliaires, d'excipients cosmétiques ou de mélanges d'entre eux, moyennant quoi toutes les indications en poids se rapportent au poids total du produit cosmétique.

3. Produit cosmétique selon la revendication 1 ou 2, **caractérisé en ce qu'**il contient des microbilles de dioxyde de silicium ayant une granulométrie D₅₀ dans une plage de 3,5 à 8 µm.

4. Produit cosmétique selon l'une des revendications 1 à 3, **caractérisé en ce que** les microbilles à base de dioxyde de silicium sont des microbilles déshydratées du type de diméthyl silylate de silice.

5. Produit cosmétique selon l'une des revendications 1 à 4, **caractérisé en ce que** le rapport en poids du dioxyde de silicium silylé à la protéine de blé est dans une plage de 1 à 3 : 1.

6. Produit cosmétique selon l'une des revendications 1 à 4, **caractérisé en ce que** le rapport en poids du dioxyde de silicium silylé à l'acide hyaluronique ou de son sel alcalin est dans une plage de 8 à 16 : 1.

7. Produit cosmétique selon l'une des revendications 1 à 6, **caractérisé en ce qu'**il contient 5 à 10 % en poids d'un alcool polyvalent.

8. Produit cosmétique selon l'une des revendications 1 à 7, **caractérisé en ce qu'**il contient un capteur de radicaux ou un mélange de capteurs de radicaux.

9. Produit cosmétique selon la revendication 8, **caractérisé en ce que** le mélange de capteurs de radicaux est présent sous forme d'extraits de plantes.

10. Produit cosmétique selon la revendication 9, **caractérisé en ce que** les extraits de plantes sont sélectionnés parmi le groupe comprenant Angelica archangelica, Arnica montana, Camellia sinensis, Cupressus semper, Coffea arabica, Polygonatum multiflorum, Pongamia pinnata, Rosmarinus officinalis, Evernia furfuracea, Evernia prunastri, Avena sativa et des mélanges d'entre eux.

11. Produit cosmétique selon l'une des revendications 1 à 10, **caractérisé en ce qu'**il contient un oligopeptide, notamment du céramide 2.
